# EUROPÄISCHE PATENTSCHRIFT

(11) **EP 2 801 330 B1**
(45) Veröffentlichungstag und Bekanntmachung des Hinweises auf die Patenterteilung: **04.07.2018**
(21) Anmeldenummer: 13167045.7
(22) Anmeldetag: 08.05.2013
(51) Int. Cl.: A61B 17/80, A61B 17/86

(54) **Knochenplatte mit einem Inlay und Verfahren zum Herstellen der Knochenplatte**
Bone plate with an inlay and method for producing the bone plate
Plaque d'ostéosynthèse dotée d'un inlay et procédé de fabrication de la plaque d'ostéosynthèse

(43) Veröffentlichungstag der Anmeldung: 12.11.2014
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll

(56) Entgegenhaltungen:
- WO-A1-2012/095720
- DE-A1- 19 629 011
- US-A1- 2004 073 218
- US-A1- 2012 071 875
- US-A1- 2013 053 847

## Beschreibung

Die Erfindung betrifft eine Knochenplatte mit einem Trägerteil und einem in das Trägerteil eingesetzten Inlay, wobei das Inlay mit dem Trägerteil verbunden ist. Das Inlay besteht aus einem weicheren Material als das Trägerteil. In dem Inlay ist genau ein Durchgangsloch ausgebildet, welches mittig in dem Inlay angeordnet ist. Die Erfindung betrifft außerdem ein Verfahren zum Herstellen einer solchen Knochenplatte
Solche Knochenplatten können im Rahmen einer chirurgischen Operation mit einem Knochen verbunden werden. Sie werden beispielsweise verwendet, um einen Knochen nach einem Bruch zu stabilisieren. Dazu wird die Knochenplatte so positioniert, dass sie sich über die Bruchstelle hinweg erstreckt, und dann an den Knochenfragmenten befestigt. Die Bruchstelle wird dadurch ruhig gestellt und der Knochen kann ausheilen. Die Knochenplatte kann auch anderen Zwecken dienen und beispielsweise ein mit einem Knochen zu verbindendes Element einer Endoprothese sein.

Zur Befestigung der Knochenplatte an dem Knochen wird eine Knochenschraube in das Durchgangsloch eingesetzt, die sowohl am Schaft als auch am Kopf mit einem Gewinde versehen ist. Der Schaft der Schraube dringt so weit in das Knochenmaterial ein, bis der Schraubenkopf in das Durchgangsloch des Inlays eintritt. Der Kopf der Knochenschraube hat einen äußeren Durchmesser, der größer ist als der kleinste Durchmesser des Durchgangslochs. Beim weiteren Eindrehen der Knochenschraube kommt der Kopf der Knochenschraube in Kontakt mit dem das Durchgangsloch umgebenden Material des Inlays. Das weiche Material des Inlays verformt sich unter dem Eingriff der Knochenschraube, so dass sich eine Gewindeverbindung zwischen dem Kopf der Knochenschraube und dem Material des Inlays bildet.

Durch die Gewindeverbindung zwischen dem Schraubenkopf und dem Durchgangsloch wird die Knochenschraube winkelstabil mit der Knochenplatte verbunden. Das System erhält dadurch eine hohe Stabilität. Die Lasten, die zwischen dem Schaft der Knochenschraube und dem Knochenmaterial übertragen werden, vermindern sich. Da die Gewindeverbindung zwischen dem Kopf der Knochenschraube und der Knochenplatte erst beim Eindrehen der Knochenschraube durch einen Umformvorgang gebildet wird, ist es nicht erforderlich, die Knochenschraube in einem bestimmten vorgegebenen Winkel in die Knochenplatte einzudrehen. Vielmehr kann der Winkel innerhalb bestimmter Grenzen frei gewählt werden. Dies gibt dem Chirurgen eine hohe Flexibilität während der Operation. Solche Knochenplatten werden auch als multidirektional winkelstabil bezeichnet.

Eine Knochenplatte mit einem Inlay aus einem weicheren Material ist bekannt, DE 196 29 011. Die Inlays sind bislang so gestaltet, dass sie im Wesentlichen kreisförmig sind und ein zentral angeordnetes Durchgangsloch aufweisen. Der Übergangsbereich zwischen dem Inlay und dem Trägerteil der Knochenplatte erstreckt sich um das Durchgangsloch herum und ist etwa konzentrisch zu dem Durchgangsloch. Für den Kraftfluss innerhalb der Knochenplatte ist ein solcher zu dem Durchgangsloch konzentrischer Übergangsbereich ungünstig.

Der Erfindung liegt die Aufgabe zu Grunde, eine Knochenplatte sowie ein Verfahren zum Herstellen einer Knochenplatte vorzustellen, die einen gleichmäßigeren Kraftfluss innerhalb der Knochenplatte ermöglichen. Ausgehend vom genannten Stand der Technik wird die Aufgabe gelöst mit den Merkmalen der Ansprüche 1 und 13. Vorteilhafte Ausführungsformen sind in den Unteransprüchen angegeben.

Erfindungsgemäß hat das Inlay in einer Längsrichtung eine größere Ausdehnung als in einer Querrichtung. Die Strecke von dem Durchgangsloch zum nächstgelegenen Ende des Inlays in Längsrichtung ist größer als die Strecke von dem Durchgangsloch zum Rand des Inlays in Querrichtung.

Durch die erfindungsgemäße Gestaltung wird es möglich, das Inlay besser in den Kraftfluss in der Knochenplatte zu integrieren. Die Längsrichtung des Inlays kann zu diesem Zweck parallel ausgerichtet werden zu der Richtung, in der die größten Kräfte innerhalb der Knochenplatte übertragen werden. Es wird dadurch vermieden, dass der Übergangsbereich zwischen dem Inlay und dem Trägerteil der Knochenplatte sich in unmittelbarer Nachbarschaft zu dem Durchgangsloch quer zum Kraftfluss erstreckt.

Die Längsrichtung des Inlays bezeichnet die Richtung, in der das Inlay die größte Ausdehnung hat. Die Querrichtung schließt einen Winkel von 90° mit der Längsrichtung ein. Die Ausdehnung des Inlays in Längsrichtung wird auch als Länge des Inlays bezeichnet, die Ausdehnung in Querrichtung als Breite. Die von der Längsrichtung und der Querrichtung aufgespannte Fläche ist die Ebene des Inlays. Die Ausdehnung des Inlays in einer dazu senkrechten Richtung wird als Dicke bezeichnet. Das Inlay hat eine Oberseite und eine Unterseite, die im Wesentlichen parallel zur Ebene des Inlays ausgerichtet sind. Das Durchgangsloch erstreckt sich von der Oberseite bis zur Unterseite des Inlays.

Von Vorteil für den Kraftfluss ist es, wenn sich das Inlay von dem Durchgangsloch in Richtung des in Längsrichtung nächstgelegenen Endes zunächst zügig und dann langsamer verjüngt. Das Verjüngen bezieht sich auf die Querrichtung des Inlays. Vorzugsweise umfasst die Kontur des Inlays in der von der Längsrichtung und der Querrichtung aufgespannten Ebene einen konkaven Abschnitt. Konkaver Abschnitt bedeutet, dass eine Verbindungsstrecke, die zwei Punkte des Abschnitts gerade miteinander verbindet, außerhalb des Inlays liegt. Der konkave Abschnitt der Kontur ist vorzugsweise in dem Bereich des Inlays angeordnet, der sich zwischen dem Durchgangsloch und dem in Längsrichtung nächstgelegenen Ende erstreckt.

Das Inlay kann so gestaltet sein, dass die Breite des Inlays sich von dem Durchgangsloch in Richtung des in Längsrichtung nächstgelegenen Endes kontinuierlich verjüngt. Das Inlay wird dann bezogen auf die Querrichtung kontinuierlich schmaler, je näher man dem Ende der Knochenplatte kommt. Alternativ ist es auch möglich, dass das Inlay zwischen dem Durchgangsloch und dem Ende eine Verbreiterung aufweist. Dies bedeutet, dass es zwischen der Verbreiterung und dem Durchgangsloch einen Bereich mit geringerer Breite gibt.

Für die Einbindung des Inlays in den Kraftfluss der Knochenplatte ist es von Vorteil, wenn das Inlay am Ende spitz zuläuft. Allerdings ist es bei einem spitzen Ende nicht ganz einfach, die Verbindung zwischen dem Inlay und dem Trägerteil der Knochenplatte herzustellen. Die Erfindung umfasst deswegen alternative Ausführungsformen, bei denen das Ende des Inlays abgerundet ist.

Die Konturfläche des Inlays, die sich um das Inlay herum erstreckt, kann rechtwinklig zur Ebene des Inlays ausgerichtet sein. Dies ermöglicht eine einfache Fertigung des Inlays. Für den Kraftfluss innerhalb der Knochenplatte ist es von Vorteil, wenn die Konturfläche des Inlays keinen rechten Winkel mit der Ebene des Inlays einschließt. Beispielsweise kann die Konturfläche bezogen auf die senkrecht zur Ebene des Inlays ausgerichtete Richtung gewölbt sein oder einen Winkel mit dieser Richtung einschließen.

Als Ausgangsmaterial für die Herstellung des Inlays kann ein durch Walzen hergestelltes Blech verwendet werden. Vorzugsweise wird das Inlay so hergestellt, dass die Längsrichtung des Inlays parallel zur Walzrichtung ist. Auch das Trägerteil der Knochenplatte kann aus einem gewalzten Blech gefertigt sein. In einer vorteilhaften Ausführungsform stimmt die Walzrichtung des Inlays mit der Walzrichtung des Trägerteils überein.

Für die Herstellung der erfindungsgemäßen Knochenplatte sind Titanmaterialien geeignet. Bei unlegiertem Titan (Reintitan) hängt die Härte bekanntlich vom Sauerstoffgehalt ab. Je höher der Sauerstoffgehalt desto größer ist die Härte. Das Inlay kann aus Reintitan geringer Härte hergestellt sein. Es kann beispielsweise Reintitan Grade 0 oder Grade 1 verwendet werden, bei dem der Sauerstoffgehalt beispielsweise kleiner als 0,2 %, vorzugsweise kleiner als 0,1 % sein kann.

Das Trägerteil der Knochenplatte kann aus Reintitan von größerer Härte gefertigt sein. Beispielsweise kann das Trägerteil aus Reintitan Grade 4 bestehen. Der Sauerstoffgehalt des Reintitan kann beispielsweise zwischen 0,3 % und 0,4 % liegen. Möglich ist es auch, das Trägerteil aus einer Titanlegierung herzustellen. Titanlegierungen haben regelmäßig eine größere Härte als Reintitan. In Betracht kommt beispielsweise TiAl6V4 oder eine Titan-Niob-Legierung. Bevor die Knochenplatte beim Patienten eingesetzt werden kann, muss das Inlay so stabil mit dem Trägerteil der Knochenplatte verbunden sein, dass das Inlay sich unter den auftretenden Kräften nicht lösen kann. Eine hinreichend stabile Verbindung zwischen dem Inlay und dem Trägerteil lässt sich beispielsweise durch Laserschweißen herstellen. Durch das Laserschweißen ergibt sich eine homogene Verbindung der Materialien. Die Verbindung erstreckt sich vorzugsweise über die gesamte Dicke der Knochenplatte. Der Bereich der Knochenplatte, in dem beim Verbinden des Inlays mit dem Trägerteil auf das Material eingewirkt wird, wird als Übergangsbereich bezeichnet.

Das Inlay kann so gestaltet sein, dass die Konturfläche flächig auf einer Gegenfläche des Trägerteils aufliegt, bevor beide Teile miteinander verbunden sind. Das Trägerteil kann zu diesem Zweck eine zu dem Inlay passende Ausnehmung aufweisen. Der flächige Kontakt zwischen der Konturfläche und der Gegenfläche erstreckt sich vorzugsweise über den gesamten Umfang des Inlays. Bezogen auf die zu der Ebene des Inlays senkrechte Richtung kann der flächige Kontakt sich über die gesamte Dicke des Inlays erstrecken. Für das Herstellen der Schweißverbindung kann es von Vorteil sein, wenn der flächige Kontakt sich nur über einen Teil der Dicke erstreckt.

Die Knochenplatte kann eine Mehrzahl von Durchgangslöchern aufweisen. Es ist möglich, dass ein Teil der Durchgangslöcher in dem Trägerteil der Knochenplatte ausgebildet ist. In einer vorteilhaften Ausführungsform sind alle Durchgangslöcher in dem weicheren Inlay-Material angeordnet. Die Knochenplatte weist für jedes Durchgangsloch ein eigenes Inlay auf. Der Kraftfluss in einer Knochenplatte wird dadurch bestimmt, an welchen Stellen der Knochenplatte die Schrauben angeordnet sind, mit denen die Verbindung zum Knochen hergestellt wird. Eine bevorzugte Richtung des Kraftflusses erstreckt sich von einer Knochenschraube zu einer benachbarten Knochenschraube. Bei der erfindungsgemäßen Knochenplatte kann dies berücksichtigt werden, indem die Längsrichtung des Inlays parallel ausgerichtet wird zu der Verbindungsstrecke zwischen dem Durchgangsloch des Inlays und einem benachbarten Durchgangsloch der Knochenplatte.

Die Erfindung betrifft außerdem ein System aus einer solchen Knochenplatte und einer Knochenschraube. Der Kopf der Schraube ist mit einem Gewinde versehen (Kopfgewinde), das dazu ausgelegt ist, das das Durchgangsloch umgebende Material des Inlays umzuformen, um eine Gewindeverbindung zu bilden. Für eine Mehrzahl von Durchgangslöchern der Knochenplatte kann das System eine Mehrzahl von Knochenschrauben umfassen. Da die Gewindeverbindung erst beim Eindrehen der Knochenschraube gebildet wird, kann die Knochenschraube unter verschiedenen Winkeln (winkelvariabel) mit der Knochenplatte verbunden werden.

In einer vorteilhaften Ausführungsform ist in dem Durchgangsloch eine Materiallippe ausgebildet, die dazu bestimmt ist, von dem Kopfgewinde der Knochenschraube umgeformt zu werden. Eine Materiallippe bezeichnet einen Bereich geringerer Materialstärke, der von der Wand des Durchgangslochs in Richtung Zentrum des Durchgangslochs vorspringt. Die Lippe kann sich über den gesamten Umfang des Durchgangslochs oder über einen Teil des Umfangs des Durchgangslochs erstrecken.

Die Erfindung betrifft außerdem ein Verfahren zum Herstellen einer solchen Knochenplatte. Bei dem Verfahren werden ein Trägerteil einer Knochenplatte und ein Inlay bereitgestellt, wobei das Inlay genau ein Durchgangsloch aufweist, welches mittig in dem Inlay angeordnet ist, und aus einem weicheren Material besteht als das Trägerteil. Das Trägerteil ist mit einer zu dem Inlay passenden Ausnehmung versehen. Das Inlay wird in die Ausnehmung eingesetzt, so dass eine Konturfläche des Inlays flächig an dem Trägerteil anliegt. Das Inlay wird entlang der Konturfläche mit dem Trägerteil verbunden. Erfindungsgemäß hat das Inlay in einer Längsrichtung eine größere Ausdehnung als in einer Querrichtung. Die Strecke von dem Durchgangsloch zum nächstgelegenen Ende des Inlays in Längsrichtung ist größer als die Strecke von dem Durchgangsloch zum nächstgelegenen Ende des Inlays in Querrichtung.

Vorzugsweise wird die Verbindung mittels Laserschweißen hergestellt. Das Verfahren kann mit weiteren Merkmalen fortgebildet werden, die im Zusammenhang der erfindungsgemäßen Knochenplatte beschrieben sind. Die Erfindung betrifft außerdem eine Knochenplatte, die mit dem erfindungsgemäßen Verfahren hergestellt werden kann.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine Ansicht von oben auf eine erfindungsgemäße Knochenplatte;
- Fig. 2:: einen Schnitt entlang Linie I-I in Fig. 1;
- Fig. D:: einen vergrößerten Ausschnitt einer Knochenplatte in einer Ansicht von oben;
- Fig. 4:: die Ansicht aus Fig. 3 bei einer anderen Ausführungsform der Erfindung;
- Fig. 5:: die Ansicht aus Fig. 2 bei einer anderen Ausführungsform der Erfindung;
- Fig. 6:: die Ansicht aus Fig. 2 bei einer weiteren Ausführungsform der Erfindung; und
- Fig. 7:: eine Knochenschraube eines erfindungsgemäßen Systems.

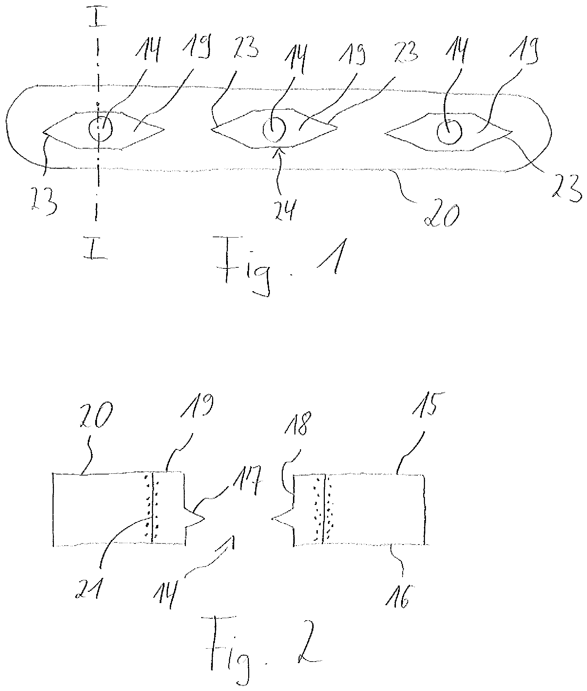

Eine in den Figuren 1 und 2 gezeigte erfindungsgemäße Knochenplatte hat drei Durchgangslöcher 14, die sich von einer Oberseite 15 der Knochenplatte bis zur einer Unterseite 16 der Knochenplatte erstrecken. In jedem Durchgangsloch 14 ist eine Lippe 17 ausgebildet, die von der Mantelfläche 18 in Richtung des Zentrums des Durchgangslochs 14 vorspringt. Die Lippe 17 ist eine durchgehende Lippe, die sich ohne Unterbrechung über den gesamten Umfang des Durchgangslochs erstreckt. Die Lippe 17 ist im unteren Bereich des Durchgangslochs 14 angeordnet, so dass oberhalb der Lippe 17 Raum für die Aufnahme des Schraubenkopfs bleibt.

Die Knochenplatte ist dazu bestimmt, mit der Unterseite 16 auf einen Knochen aufgelegt zu werden, so dass die Knochenplatte sich über eine Bruchstelle des Knochens hinweg erstreckt. Nachdem der Knochen in die richtige Stellung reponiert ist, wird die Knochenplatte mit den Knochenfragmenten verbunden. Der Knochen ist dann in dieser Stellung fixiert und kann ausheilen.

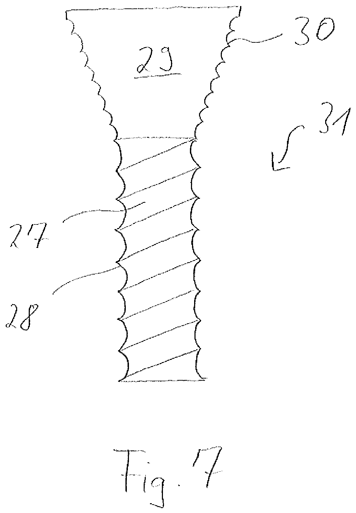

Zum Verbinden der Knochenplatte mit dem Knochen werden Knochenschrauben 31 verwendet, wie sie in Fig. 7 dargestellt sind. Die Knochenschrauben 31 haben einen Schaft 27, der mit einem Knochengewinde 28 versehen ist, und einen Schraubenkopf 29, dessen Außenfläche ein Kopfgewinde 30 mit kleinerer Steigung aufweist. Die Mantelfläche des Schraubenkopfs 29 schließt mit der Schraubenachse einen Winkel von 25° ein. Die Knochenschraube wird in den Knochen eingedreht, bis der Schraubenkopf 29 in das Durchgangsloch 14 eindringt. Dabei kann der Winkel, den die Knochenschraube mit der Achse des Durchgangslochs einschließt, zwischen etwa 0° und 15° frei gewählt werden. Wenn die Knochenschraube nun weiter eingedreht wird, kommt das Kopfgewinde 30 der Knochenschraube 31 in Eingriff mit der Lippe 17. Die Lippe 17 wird verformt und es wird eine Gewindeverbindung zwischen dem Kopf 29 der Knochenschraube 31 und der Lippe 17 gebildet.

Bei der in Fig. 1 gezeigten Knochenplatte sind die drei Durchgangslöcher 14 jeweils in einem Inlay 19 ausgebildet. Das Inlay besteht aus Reintitan Grade 1 oder Grade 0 und damit aus einem Material vergleichsweise geringer Härte.

Die Inlays sind eingesetzt in passende Ausnehmungen eines Trägerteils 20 der Knochenplatte. Das Trägerteil 20 besteht aus Reintitan Grade 4 oder aus der Titanlegierung TiAl6V4. Die Konturfläche des Inlays 19, die in Fig. 2 mit einer durchgezogenen Linie angedeutet ist, ist senkrecht zu der Ebene des Inlays 19 ausgerichtet. Die Konturfläche liegt über die gesamte Dicke der Knochenplatte flächig auf der Gegenfläche des Trägerteils 20 auf.

Mit der durchgezogenen Linie der Konturfläche ist der Zwischenzustand der Knochenplatte angedeutet, in dem das Inlay 19 in das Trägerteil 20 eingesetzt ist, die endgültige Verbindung jedoch noch nicht hergestellt ist. Verbunden wird das Inlay 19 mit dem Trägerteil 20 durch Laserschweißen. Durch das Laserschweißen bildet sich eine weitgehend homogene Verbindung zwischen dem Material des Inlays 19 und dem Material des Trägerteils 20. Der Übergangsbereich zwischen dem Inlay 19 und dem Übergangsteil 20 ist in Fig. 2 punktiert dargestellt.

Die Knochenschraube 31 besteht aus der Titanlegierung Ti-Al6V4 und damit aus einem härteren Material als das Inlay 19. Kommt der Kopf 29 der Knochenschraube in Eingriff mit der Lippe 17, verformt sich die Lippe 17 und es entsteht eine Gewindeverbindung zwischen dem Kopfgewinde 30 und der Lippe 17. Die Knochenschraube 31 wird dadurch winkelstabil mit der Knochenplatte verbunden.

Wie Fig. 1 zeigt, hat das Inlay 19 in Längsrichtung eine größere Ausdehnung als in Querrichtung. Die Längsrichtung des Inlays 19 stimmt mit der Längsrichtung der Knochenplatte überein. Außerdem fällt die Längsrichtung des Inlays 19 mit der Linie zusammen, die die drei Durchgangslöcher 14 verbindet. Das Rohmaterial für die Inlays 19 sowie für das Trägerteil 20 bildet jeweils ein gewalztes Blech. Sowohl bei dem Inlay 19 als auch bei dem Trägerteil 20 stimmt die Walzrichtung mit der Längsrichtung überein.

Die Kontur des Inlays 19 umfasst zwei in Längsrichtung ausgerichtete Abschnitte, die das Durchgangsloch 14 zwischen sich einschließen. Zu den beiden in Längsrichtung gelegenen Enden 23 hin verjüngt sich die Kontur des Inlays 19 kontinuierlich und läuft am Ende spitz zu. Das Durchgangsloch 14 ist mittig in dem Inlay 19 angeordnet. Die Strecke von dem Durchgangsloch 14 zum Rand 24 des Inlays 19 in Querrichtung ist jeweils kürzer als die Strecke zu dem in Längsrichtung gelegenen Ende 23 des Inlays 19.

Die Längsrichtung der Inlays 19 entspricht der Richtung, in der die größten Kräfte innerhalb der Knochenplatte übertragen werden. Die Kontur des Inlays 19 ist also entweder parallel zu der Hauptrichtung des Kraftflusses oder schließt einen spitzen Winkel mit dieser Richtung ein. Dadurch wird eine gleichmäßige Kraftübertragung innerhalb der Knochenplatte erreicht.

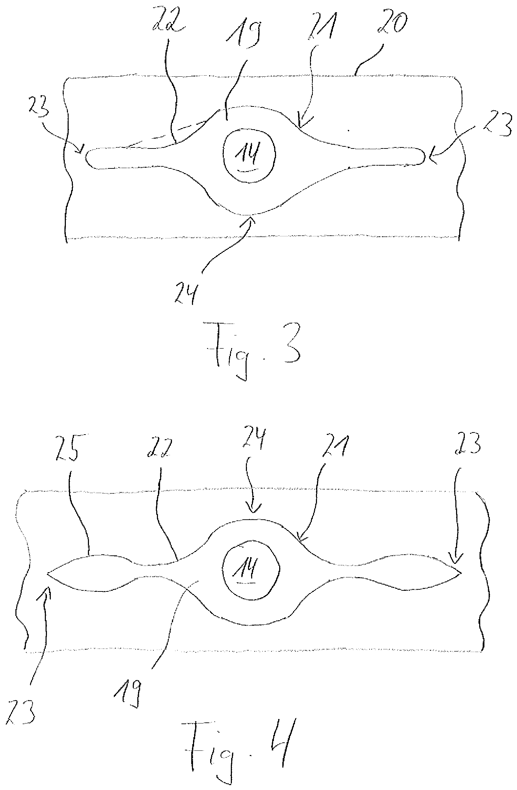

Bei der Ausführungsform gemäß Fig. 3 hat das Inlay 19 ebenfalls in Längsrichtung eine größere Ausdehnung als in Querrichtung. Die Kontur 21 des Inlays 19 hat einen konkaven Abschnitt 22. In Fig. 3 ist dies mit einer gestrichelten Linie angedeutet, die zwei Punkte der Kontur 21 verbindet und die außerhalb des Inlays 19 liegt. Es hat sich gezeigt, dass der Kraftfluss innerhalb der Knochenplatte durch solche konkaven Abschnitte in der Kontur 21 des Inlays 19 verbessert werden kann.

An den Enden 23 ist das Inlay 19 abgerundet. Man nimmt damit in Kauf, dass die Kontur 21 des Inlays 19 die Richtung des Kraftflusses rechtwinklig schneidet. Allerdings ist es bei einer solchen Gestaltung einfacher, den für einen gleichmäßigen Kraftfluss vorteilhaften homogenen Material-übergang zwischen dem Material des Inlays 19 und dem Material des Trägerteils 20 herzustellen.

In Fig. 4 schließt sich an den konkaven Abschnitt 22 in der Kontur 21 des Inlays 19 eine Verbreiterung 25 an. An den Enden 23 läuft das Inlay 19 spitz zu. Durch die Verbreiterung 25 verlängert sich die Kontur 21 des Inlays 19, was sich als vorteilhaft für einen gleichmäßigen Kraftfluss innerhalb der Knochenplatte erwiesen hat.

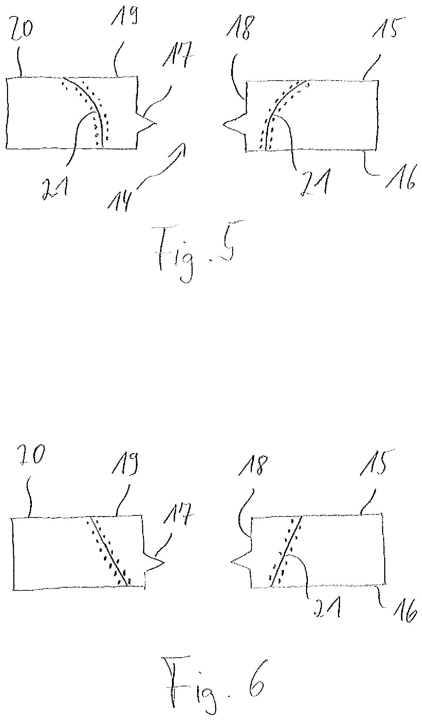

Bei den Ausführungsformen der Figuren 5 und 6 ist die Konturfläche nicht senkrecht zu der Ebene des Inlays 19 ausgerichtet, sondern das Inlay 19 ist am unteren Ende schmaler und weitet sich nach oben hin auf. In Fig. 5 ist der Übergang von der Unterseite 16 zur Oberseite 15 gewölbt, was für einen gleichmäßigen Kraftfluss innerhalb der Knochenplatte von Vorteil ist. In Fig. 6 ist die Konturfläche eben und schließt einen Winkel mit der Vertikalen ein. Bei einer ebenen Konturfläche ist es einfacher, durch Laserschweißen einen homogenen Übergang zwischen dem Material des Inlays 19 und dem Material des Trägerteils 20 zu schaffen.

## Patentansprüche

1. Knochenplatte mit einem Trägerteil (20) und einem in das Trägerteil (20) eingesetzten und mit dem Trägerteil (20) verbundenen Inlay (19), wobei das Inlay (19) aus einem weicheren Material besteht als das Trägerteil (20) und wobei in dem Inlay (19) genau ein Durchgangsloch (14) ausgebildet ist, welches mittig in dem Inlay (19) angeordnet ist, **dadurch gekennzeichnet, dass** das Inlay (19) in einer Längsrichtung eine größere Ausdehnung hat als in einer Querrichtung und dass die Strecke von dem Durchgangsloch (14) zum nächstgelegenen Ende (23) des Inlays (19) in Längsrichtung größer ist als die Strecke von dem Durchgangsloch (14) zum Rand (24) des Inlays (19) in Querrichtung.

2. Knochenplatte nach Anspruch 1, **dadurch gekennzeichnet, dass** die Kontur (21) des Inlays (19) einen konkaven Abschnitt (22) aufweist.

3. Knochenplatte nach Anspruch 1 oder 2, **dadurch gekennzeichnet, dass** die Breite des Inlays (19) sich von dem Durchgangsloch (14) in Richtung des in Längsrichtung nächstgelegenen Endes (23) kontinuierlich verjüngt.

4. Knochenplatte nach Anspruch 1 oder 2, **dadurch gekennzeichnet, dass** das Inlay (19) zwischen dem Durchgangsloch (14) und dem in Längsrichtung nächstgelegenen Ende (23) eine Verbreiterung (25) aufweist.

5. Knochenplatte nach einem der Ansprüche 1 bis 4,
**dadurch gekennzeichnet, dass** das in Längsrichtung nächstgelegene Ende (23) des Inlays (19) spitz zuläuft.

6. Knochenplatte nach einem der Ansprüche 1 bis 4,
**dadurch gekennzeichnet, dass** das in Längsrichtung nächstgelegene Ende (23) des Inlays (19) abgerundet ist.

7. Knochenplatte nach einem der Ansprüche 1 bis 6,
**dadurch gekennzeichnet, dass** eine Konturfläche des Inlays (19) bezogen auf die senkrecht zur Ebene des Inlays (19) ausgerichtete Richtung gewölbt ist oder einen Winkel mit dieser Richtung einschließt.

8. Knochenplatte nach einem der Ansprüche 1 bis 7,
**dadurch gekennzeichnet, dass** das Inlay (19) aus gewalztem Blech gefertigt ist und dass die Walzrichtung parallel zur Längsrichtung des Inlays (19) ist.

9. Knochenplatte nach einem der Ansprüche 1 bis 8,
**dadurch gekennzeichnet, dass** das Inlay (19) aus Reintitan Grade 0 oder Grade 1 besteht.

10. Knochenplatte nach einem der Ansprüche 1 bis 9,
**dadurch gekennzeichnet, dass** das Trägerteil (20) aus Reintitan Grade 4 oder aus einer Titanlegierung besteht.

11. Knochenplatte nach einem der Ansprüche 1 bis 10,
**dadurch gekennzeichnet, dass** die Knochenplatte ein zweites Durchgangsloch (14) aufweist und dass die Längsrichtung des Inlays (19) parallel ausgerichtet ist zu der Verbindungsstrecke zwischen dem Durchgangsloch (14) des Inlays (19) und dem zweiten Durchgangsloch (14).

12. System aus einer Knochenplatte gemäß einem der Ansprüche 1 bis 11 und einer Knochenschraube (31), wobei die Knochenschraube (31) ein Kopfgewinde (30) aufweist, das dazu ausgelegt ist, das Material des Inlays (19) umzuformen, um eine Gewindeverbindung zu bilden

13. Verfahren zum Herstellen einer Knochenplatte, bei dem ein Trägerteil (20) der Knochenplatte und ein Inlay (19) bereitgestellt werden, wobei das Inlay (19) genau ein Durchgangsloch (14) aufweist, welches mittig in dem Inlay angeordnet ist, und aus einem weicheren Material besteht als das Trägerteil (20) und wobei das Trägerteil (20) mit einer zu dem Inlay (19) passenden Ausnehmung versehen ist, bei dem das Inlay (19) in die Ausnehmung eingesetzt wird, so dass eine Konturfläche des Inlays (19) flächig an dem Trägerteil (20) anliegt, **dadurch gekennzeichnet, dass** das Inlay (19) entlang der Konturfläche mit dem Trägerteil (20) verbunden wird, wobei das Inlay (19) in einer Längsrichtung eine größere Ausdehnung hat als in einer Querrichtung und wobei die Strecke von dem Durchgangsloch (14) zum nächstgelegenen Ende (23) des Inlays (19) in Längsrichtung größer ist als die Strecke von dem Durchgangsloch (14) zum Rand des Inlays (19) in Querrichtung.

14. Verfahren nach Anspruch , **dadurch gekennzeichnet, dass** das Inlay (19) durch Laserschweißen mit dem Trägerteil (20) verbunden wird.

## Claims

1. Bone plate having a carrier part (20) and an inlay (19) which is inserted into the carrier part (20) and connected to the carrier part (20), wherein the inlay (19) consists of a softer material than the carrier part (20) and wherein the inlay (19) has formed therein precisely one through-hole (14) which is arranged centrally in the inlay (19), **characterized in that** the inlay (19) has a greater extent in a longitudinal direction than in a transverse direction, and **in that** the distance from the through-hole (14) to the closest end (23) of the inlay (19) in the longitudinal direction is greater than the distance from the through-hole (14) to the edge (24) of the inlay (19) in the transverse direction.

2. Bone plate according to Claim 1, **characterized in that** the contour (21) of the inlay (19) has a concave portion (22).

3. Bone plate according to Claim 1 or 2, **characterized in that** the width of the inlay (19) continuously tapers from the through-hole (14) in the direction of the end (23) which is closest in the longitudinal direction.

4. Bone plate according to Claim 1 or 2, **characterized in that** the inlay (19) has a widening (25) between the through-hole (14) and the end (23) which is closest in the longitudinal direction.

5. Bone plate according to one of Claims 1 to 4, **characterized in that** the end (23) of the inlay (19) that is closest in the longitudinal direction tapers to a point.

6. Bone plate according to one of Claims 1 to 4, **characterized in that** the end (23) of the inlay (19) that is closest in the longitudinal direction is rounded off.

7. Bone plate according to one of Claims 1 to 6, **characterized in that** a contoured surface of the inlay (19) is curved with respect to the direction oriented perpendicularly to the plane of the inlay (19) or encloses an angle with this direction.

8. Bone plate according to one of Claims 1 to 7, **characterized in that** the inlay (19) is produced from rolled sheet metal, and **in that** the rolling direction is parallel to the longitudinal direction of the inlay (19).

9. Bone plate according to one of Claims 1 to 8, **characterized in that** the inlay (19) consists of pure titanium Grade 0 or Grade 1.

10. Bone plate according to one of Claims 1 to 9, **characterized in that** the carrier part (20) consists of pure titanium Grade 4 or of a titanium alloy.

11. Bone plate according to one of Claims 1 to 10, **characterized in that** the bone plate has a second through-hole (14), and **in that** the longitudinal direction of the inlay (19) is oriented parallel to the path connecting the through-hole (14) of the inlay (19) and the second through-hole (14).

12. System consisting of a bone plate according to one of Claims 1 to 11 and a bone screw (31), wherein the bone screw (31) has a head thread (30) which is designed to deform the material of the inlay (19) in order to form a threaded connection.

13. Method for producing a bone plate, in which a carrier part (20) of the bone plate and an inlay (19) are provided, wherein the inlay (19) has precisely one through-hole (14) which is arranged centrally in the inlay, and consists of a softer material than the carrier part (20), and wherein the carrier part (20) is provided with a cutout matching the inlay (19), wherein the inlay (19) is inserted into the cutout such that a contoured surface of the inlay (19) bears flat against the carrier part (20), **characterized in that** the inlay (19) is connected to the carrier part (20) along the contoured surface, wherein the inlay (19) has a greater extent in a longitudinal direction than in a transverse direction, and wherein the distance from the through-hole (14) to the closest end (23) of the inlay (19) in the longitudinal direction is greater than the distance from the through-hole (14) to the edge of the inlay (19) in the transverse direction.

14. Method according to Claim , **characterized in that** the inlay (19) is connected to the carrier part (20) by laser welding.

## Revendications

1. Plaque d'ostéosynthèse dotée d'une partie de support (20) et d'un inlay (19) inséré dans la partie de support (20) et assemblé à la partie de support (20), dans laquelle l'inlay (19) se compose d'un matériau plus mou que la partie de support (20) et dans laquelle exactement un trou de passage (14), qui est disposé au milieu dans l'inlay (19), est formé dans l'inlay (19), **caractérisée en ce que** l'inlay (19) présente une plus grande extension dans une direction longitudinale que dans une direction transversale et **en ce que** la distance du trou de passage (14) à l'extrémité (23) de l'inlay (19) située le plus près en direction longitudinale est plus grande que la distance du trou de passage (14) au bord (24) de l'inlay (19) en direction transversale.

2. Plaque d'ostéosynthèse selon la revendication 1, **caractérisée en ce que** le contour (21) de l'inlay (19) présente une partie concave (22).

3. Plaque d'ostéosynthèse selon la revendication 1 ou 2, **caractérisée en ce que** la largeur de l'inlay (19) diminue de façon continue à partir du trou de passage (14) en direction de l'extrémité située le plus près (23) en direction longitudinale.

4. Plaque d'ostéosynthèse selon la revendication 1 ou 2, **caractérisée en ce que** l'inlay (19) présente un élargissement (25) entre le trou de passage (14) et l'extrémité située le plus près (23) en direction longitudinale.

5. Plaque d'ostéosynthèse selon l'une quelconque des revendications 1 à 4, **caractérisée en ce que** l'extrémité (23) de l'inlay (19) située le plus près en direction longitudinale se termine en pointe.

6. Plaque d'ostéosynthèse selon l'une quelconque des revendications 1 à 4, **caractérisée en ce que** l'extrémité (23) de l'inlay (19) située le plus près en direction longitudinale est arrondie.

7. Plaque d'ostéosynthèse selon l'une quelconque des revendications 1 à 6, **caractérisée en ce qu'**une face de contour de l'inlay (19) est bombée par rapport à la direction orientée perpendiculairement au plan de l'inlay (19) ou forme un angle avec cette direction.

8. Plaque d'ostéosynthèse selon l'une quelconque des revendications 1 à 7, **caractérisée en ce que** l'inlay (19) est fabriqué en tôle laminée et **en ce que** la direction de laminage est parallèle à la direction longitudinale de l'inlay (19).

9. Plaque d'ostéosynthèse selon l'une quelconque des revendications 1 à 8, **caractérisée en ce que** l'inlay (19) se compose de titane pur de qualité 0 ou de qualité 1.

10. Plaque d'ostéosynthèse selon l'une quelconque des revendications 1 à 9, **caractérisée en ce que** la partie de support (20) se compose de titane pur de qualité 4 ou d'un alliage de titane.

11. Plaque d'ostéosynthèse selon l'une quelconque des revendications 1 à 10, **caractérisée en ce que** la plaque d'ostéosynthèse présente un second trou de passage (14) et **en ce que** la direction longitudinale de l'inlay (19) est orientée parallèlement à la ligne de jonction entre le trou de passage (14) et le second trou de passage (14).

12. Système composé d'une plaque d'ostéosynthèse selon l'une quelconque des revendications 1 à 11 et d'une vis à os (31), dans lequel la vis à os (31) présente un filet de tête (30), qui est conçu pour déformer le matériau de l'inlay (19) afin de former un assemblage vissé.

13. Procédé de fabrication d'une plaque d'ostéosynthèse dans lequel on prépare une partie de support (20) de la plaque d'ostéosynthèse et un inlay (19), dans lequel l'inlay (19) présente exactement un trou de passage (14), qui est disposé au milieu de l'inlay (19), et se compose d'un matériau plus mou que la partie de support (20) et dans lequel la partie de support (20) est dotée d'un évidement adapté à l'inlay (19), dans lequel l'inlay (19) est inséré, de telle manière qu'une face de contour de l'inlay (19) s'applique en surface sur la partie de support (20), **caractérisé en ce que** l'on assemble l'inlay (19) à la partie de support (20) le long de la face de contour, dans lequel l'inlay (19) présente une plus grande extension dans une direction longitudinale que dans une direction transversale et dans lequel la distance du trou de passage (14) à l'extrémité (23) de l'inlay (19) située le plus près en direction longitudinale est plus grande que la distance du trou de passage (14) au bord de l'inlay (19) en direction transversale.

14. Procédé selon la revendication, **caractérisé en ce que** l'on assemble l'inlay (19) à la partie de support (20) par soudage au laser.
